# EUROPEAN PATENT SPECIFICATION

(11) **EP 2 418 599 B1**
(45) Date of publication and mention of the grant of the patent: **10.07.2019**
(21) Application number: 11188221.3
(22) Date of filing: 12.12.2007
(51) Int. Cl.: G06F 19/00, G16H 10/20

(54) **GRAPHICAL USER INTERFACES (GUI), METHODS AND APPARATUS FOR DATA PRESENTATION**
GRAFISCHE BENUTZEROBERFLÄCHE, VERFAHREN UND VORRICHTUNG ZUR DATENPRÄSENTATION
INTERFACES GRAPHIQUES UTILISATEUR, PROCÉDÉS ET APPAREIL POUR LA PRÉSENTATION DE DONNÉES

(30) Priority: 12.12.2006 US 874280 P; 30.04.2007 US 914822 P
(43) Date of publication of application: 15.02.2012
(62) Divisional of application: 07849566.0
(73) Proprietor: Dune Medical Devices Ltd., 38900 Caesarea (IL)
(72) Inventor: Hashimshony, Dan, 37808 Givat Ada (IL); Yarden, Orit, 54056 Givat Shmuel (IL); Aharonowitz, Gal, 44910 Gan Haim (IL); Cohen, Gil, P.O. Box 3131 Caesarea Industrial Park 38900 (IL)
(74) Representative: Becker Kurig Straus

(56) References cited:
- GB-A- 2 324 450
- US-A1- 2001 036 304
- US-A1- 2006 253 107
- US-A1- 2006 264 738
- US-B1- 6 409 684

## Description

### FIELD OF THE INVENTION

Embodiments of the invention relate generally to graphical user interfaces (GUI), methods and apparatus useful in presentation of data.

### BACKGROUND OF THE INVENTION

Considerable research and development has been previously conducted in the fields of tissue characterization, medical imaging and computerized surgical planning and/or guidance. The following listing of reference is presented as being generally indicative of the types of technology known to those of ordinary skill in the art. The list does not purport to be exhaustive.

Variations in electrical impedance of the human tissue are described in the patent literature to provide indications of tumors, lesions and other abnormalities. For example, U.S. Patents 4,291,708; 4,458,694; 4,537,203; 4,617,939 and 4,539,640 exemplify systems for tissue characterization by using multi-element probes which are pressed against the skin of the patient and measure impedance of the tissue to generate a two-dimensional impedance map.

Other techniques of this type are described in WO 01/43630 and in U.S. Patents 4,291,708 and 5,143,079.

U.S. Patents 5,807,257; 5,704,355 and 6,061,589 describe use of millimeter and microwave devices to measure bioimpedance and to detect abnormal tissue. These methods direct a free propagating radiation, or a guided radiation via waveguide, onto the organ. The radiation is focused on a relatively small volume inside the organ, and the reflected radiation is then measured.

U.S. Patent 6,109,270 describes a measurement concept with a multi-modality instrument for tissue identification in real-time neuro-surgical applications.

U.S. Patents 6,813,515; 7082325 and 7,184,824 U.S. Patent Applications published as 20070260156; 20070255169; 20070179397; 20070032747; 20070032739; 20060264738; 20060253107; 20050021019; 20030187366 and 20030138378 describe tools systems and methods useful in assessing tissue type and/or identifying tumor margins.

U.S. Patent 5,615,132 describes a method and apparatus for determining position and orientation of a moveable object using accelerometers.

U.S. Patent 6,833,814 describes an intrabody navigation system for medical applications in which three planar antennas that at least partly overlap are used to transmit electromagnetic radiation simultaneously.

Ascension Technology Corp. (Burlington VT, USA) markets a guide for localizing medical instruments with 3D magnetic tracking as "3D Guidance ^{Tm} Medsafe" .

### GENERAL DESCRIPTION

A broad aspect of the invention relates to integrating image and/or data (which may be biological, chemical and/or physical data) with planning and/or execution of a procedure. In those embodiments of the invention which are medically oriented, the image data may be, for example, X-ray based (e.g. Fluorography, or computerized tomography [CT]), ultrasound based or magnetic resonance induction (MRI) based. It should be understood that that term "image" used herein refers to data representation indicative of the properties of an examined/measured subject (or substrate) within a region of interest, e.g. tissue region. The examined/measured properties of the subject may include optical, electrical, geometrical characteristics of the subject within the region of interest. The obtained image does not necessarily presents a continuous distribution of the required property or properties all along the region of interest, but in most cases is in the form of a plurality of data pieces corresponding to discrete measurement sites within the region of interest.

According to one aspect of some embodiments of the invention, a graphical user interface (GUI) presents data in meaningful groups to a user. More specifically, the present invention deals with medical data (for example data pertaining to excised tissue) and is therefore described below with respect to this specific application. It should, however, be understood that the invention is not limited to this application and the inventive aspects can be used for various other applications as well.. In some exemplary embodiments of the invention, grouped data pertaining to excised tissue is presented to a user in an operating room, optionally during an excision procedure.

According to various embodiments of the inventions, presentation of data for a group includes a collection of individual data and/or one or more statistics which summarize the group.

Another aspect of some embodiments of the invention relates to an apparatus configured and operable to group data pertaining to individual points on a substrate into groups during data acquisition. In some exemplary embodiments of the invention, a user provides input pertaining to group definitions. Optionally, group definitions are selected from a list and/or defined by the user (i.e. "from scratch"). In some exemplary embodiments of the invention, grouped data is presented graphically on a display screen. Optionally, the grouped data is presented superimposed on a representation of the substrate. The representation of the substrate can be, for example a theoretical representation (e.g. a cube), a simplified solid form or an image of the substrate. In medical applications, common imaging modalities include, but are not limited to ultrasound imaging, fluoroscopy, computerized tomography (CT), magnetic resonance induction (MRI), impedance based and other electrical signal based measurements. In some embodiments of the invention, images are prepared concurrently with data acquisition. In other embodiments of the invention, a previously acquired image is employed. In some exemplary embodiments of the invention, decreasing an elapsed time between image acquisition and acquisition of other data to be registered onto an image contributes to an increase in accuracy of registration. Optionally group and/or positional and/or point characterization data is stored in an available memory.

An aspect of some embodiments of the invention pertains to user selection of locations on a substrate for data collection and user grouping of the locations into groups. In some exemplary embodiments of the invention, the user groups the locations into groups using an input device on a hand held data collection probe. Optionally, the substrate includes an excised tissue mass and/or a cavity from which tissue has been excised. In some exemplary embodiments of the invention, user grouping of the locations into groups is indicative of approximate positions of the locations on the substrate.

An aspect of some embodiments of the invention relates to determination of relative position between locations on a substrate and a previously placed marker. Optionally, the marker is placed during image guided localization. In some exemplary embodiments of the invention, a measurement of a relative position between a data collection probe and the marker is made substantially as data is being collected at the location. Optionally, the measurement of relative position can be made by signal strength evaluation and/or signal orientation/polarization and/or use of external position sensors and/or image analysis and/or use of overlapping planar antennas.

An aspect of some embodiments of the invention relates to a hand held device adapted to gather data from a plurality of substrate locations and group the data into user defined groups. Optionally, a user input mechanism on the device allows grouping the data into groups. Optionally, the hand held device is connectable to an external data analysis unit and/or an external vacuum pump. In some exemplary embodiments of the invention, the hand held device is sterilized and/or is provided in sterile packaging.

An aspect of some embodiments of the invention relates to presenting data indicative of substrate status on a model of the substrate. In some exemplary embodiments of the invention, a single probe gathers data on substrate status and position coordinates concurrently. In some exemplary embodiments of the invention, a user indicates a position for each data point. Optionally, the model is a predefined geometric model (e.g. a cube, tube, cylinder or sphere) or a realistic model (e.g. based on a medical image).

An aspect of some embodiments of the invention relates to integration of position data with data indicative of substrate status for planning and/or execution of a procedure on the substrate and/or surrounding material. Optionally, the position data indicates a position of a probe providing the data indicative of substrate status at the time a specific datum is acquired and/or a position of a tool. In medical embodiments of the invention, the tool can be a cutting tool and/or a sampling tool. Optionally, integration of position data with data indicative of substrate status is used in planning and/or execution of a procedure on the substrate and/or surrounding region.

In some exemplary embodiments of the invention, a graphical user interface (GUI) is provided. The GUI includes: (a) a group definition module (software and/or hardware utility) adapted to accept a user input defining groups; (b) a data receiver operable to receive a plurality of individual measurement input datum indicative of status of a substrate; (c) a grouping module (utility) configured to assign each of the individual measurement input datum to one of the groups to produce grouped data; and (d) an output module (utility) adapted to output the grouped data.

In some exemplary embodiments of the invention, an apparatus is provided. The apparatus includes: (a) a probe operable to produce a plurality of individual signal datum indicative of substrate status; (b) a group definition module adapted to accept a user input defining groups; (c) a grouping module configured to receive the signal includes the individual datum and assign each of the individual datum to one of the groups to produce grouped data; and (d) an output module adapted to output the grouped data.

In some exemplary embodiments of the invention, there is provided a method of analyzing a substrate. The method includes: (a) selecting a plurality of locations on a substrate; (b) collecting a datum indicative of substrate status at each of the locations; and (c) grouping the datum into user defined groups.

In some exemplary embodiments of the invention, there is provided a substrate analysis system. The system includes: (a) a substrate position indicator positioned at the substrate; (b) a probe operable to evaluate substrate status and output status indicative datum at individual points; (c) a measurement module configured to determine a relative position of the position indicator and the probe; and (d) a controller adapted to coordinate operation of the probe and the measurement module so that the relative position is determined for each of the individual points.

In some exemplary embodiments of the invention, there is provided a surgical supervision system. The system includes: (a) an input module adapted to receive a path from a procedure planning as described herein; and (b) a guidance system adapted to output guidance instructions to guide a surgical tool in accordance with the path.

In some exemplary embodiments of the invention, there is provided a substrate probe, the probe includes: (a) a data acquisition module adapted to engage a substrate at a user selected point, analyze the substrate and produce a datum indicative of substrate status at the point; (b) a user input device adapted to accept a user input defining groups; (c) a signal conduit adapted to relay electrical signals; and (d) a lumen adapted to relay a negative pressure from an external vacuum source to the data acquisition module.

In some exemplary embodiments of the invention, there is provided a substrate probe, the probe includes: (a) a data acquisition module adapted to engage a substrate at a user selected point, analyze the substrate and produce a datum indicative of substrate status at the point; (b) a position determination module adapted to provide a position of the point; (c) a user input device; (d) a signal conduit adapted to relay electrical signals; and (e) a lumen adapted to relay a negative pressure from an external vacuum source to the data acquisition module.

In some exemplary embodiments of the invention, there is provided an apparatus for analysis of a substrate. The apparatus includes: (a) a probe operable to produce a plurality of individual signal datum indicative of substrate status at specified locations; (b) a modeling module adapted to produce a three dimensional model of the substrate; (c) a registration module adapted to indicate the specified locations on the model; and (d) an output module adapted to present an indication of substrate status at each of the specified location on the model.

According to various exemplary embodiments of the invention, one or more of the following optional features is included.

Optionally, a registration module is included
Optionally, the registration module registers the grouped data onto an item selected from the group consisting of a solid representation of the substrate, a space filling model of the substrate and an image of the substrate (e.g. a three dimensional image of the substrate).

Optionally, the user input includes a start command and a stop command.

Optionally, the user input includes a name.

Optionally, the name is indicative of a location on the substrate.

Optionally, the names are presented in nested menus.

Optionally, the individual datum indicative of status of the substrate are linked to position coordinates.

Optionally, the position coordinates are defined relative to the substrate.

Optionally, the output of the grouped data includes the individual datum arranged in groups.

Optionally, the output of the grouped data includes at least one statistic summarizing the individual datum in each of the groups.

Optionally, a memory module adapted to store grouped data output by the output module is included.

Optionally, the data receiver is adapted to receive individual measurement input datum concurrently from an array of probes.

Optionally, the user input is provided at a time selected from before, during and after receipt of the plurality of individual measurement input datum by the data receiver.

Optionally, at least one user input mechanism on the probe is included.

Optionally, at least one indicator on the probe is included

Optionally, the output module includes a display.

Optionally, the output module includes a memory.

Optionally, the probe is adapted to measure dielectric properties of the substrate.

Optionally, the probe is adapted to measure electromagnetic properties of the substrate.

Optionally, the probe is manually operable.

Optionally, the plurality of individual datum indicative of substrate status is correlated to manually selected locations on the substrate.

Optionally, the user input is indicative of a location on the substrate.

Optionally, the probe includes a plurality of sub-probes arranged in a spatial array, the sub-probes collectively operable by a single probe activation signal.

Optionally, the probe is operable in at least two sensing/characterization modalities.

Optionally, the selecting includes visual inspection.

Optionally, the datum indicative of substrate status includes an evaluation of dielectric properties.

Optionally, the datum indicative of substrate status includes an evaluation of electromagnetic properties.

Optionally, embodiments of the invention include outputting the groups.

Optionally, embodiments of the invention include computing at least one statistic summarizing the individual datum in each of the groups and outputting the at least one statistic.

Optionally, embodiments of the invention include mapping the groups onto a representation of the substrate.

Optionally, the mapping is onto a representation of the substrate selected from the group consisting of a solid representation of the substrate, a space filling model of the substrate and an image of the substrate.

Optionally, the user defined groups are each indicative of a location on the substrate.

Optionally, embodiments of the invention include defining position coordinates for each of the locations on the substrate.

Optionally, the signal originates from the probe and is received at the position indicator.

Optionally, the signal originates from the position indicator and is received at the probe.

Optionally, embodiments of the invention include a position sensor adapted to determine a first probe position and a second substrate position indicator position; wherein the measurement module determines the relative position by comparing the first and second positions.

Optionally, a substrate analysis system according to an exemplary embodiment of the invention includes an imaging module adapted to produce an image depicting the probe and the position indicator; wherein the measurement module determines the relative position from the image.

Optionally, a substrate analysis system according to an exemplary embodiment of the invention includes a registration module adapted to register the status indicative datum from the individual points with medical image data.

Optionally, a substrate analysis system according to an exemplary embodiment of the invention includes a registration module adapted to register local summaries of status indicative datum with medical image data.

Optionally, exemplary embodiments of the invention include a cutting tool mounted on the probe.

Optionally, an exemplary embodiment of the invention includes an output module adapted to output the relative position together with the status indicative datum for each of the individual points.

Optionally, a procedure planning module is included.

Optionally, the procedure planning module is adapted to analyze the output from the output module and calculate a path.

Optionally, the procedure planning module is adapted to accept user input pertaining to a planned path.

Optionally, probes in some embodiments of the invention include a connector to an external data analysis component.

Optionally, probes in some embodiments of the invention are provided as a sterile medical device.

Optionally, the user input device is configured to group the datum into groups of data.

Optionally, a user perceptible indicator of the substrate status is provided on the probe.

Optionally, the user perceptible indicator produces an audible indication.

Optionally, the user perceptible indicator produces a visible indication.

Optionally, some embodiments of the invention are adapted to accept a user input specifying a position of the locations.

Optionally, some embodiments of the invention are adapted to determine a position of the locations.

Optionally, some embodiments of the invention include a position sensor adapted to determine a position of the locations.

Optionally, a model employed by embodiments of the invention includes a predefined geometric model.

Optionally, a model employed by embodiments of the invention includes a realistic model indicative of actual substrate shape.

Optionally, embodiments of the invention are adapted to accept medical image data as an input and generate the realistic model from the medical image data.

Optionally, the output is directed to an item selected from the group consisting of a graphic display, a printer and a memory.

The invention will be carried out according to the attached independent claims.

### BRIEF DESCRIPTION OF THE DRAWINGS

In order to understand the invention and to see how it may be carried out in practice, some embodiments are described, by way of non-limiting example only, with reference to the accompanying drawings. For simplicity and clarity of illustration, elements shown in the figures have not necessarily been drawn to scale. For example, the dimensions of some of the elements may be exaggerated relative to other elements for clarity. Further, where considered appropriate, reference numerals are repeated among the figures to indicate corresponding or analogous elements.

In the drawings:
**FIG. 1** is a schematic diagram of a system according to one exemplary embodiment of the invention;
**FIG. 2A** is a schematic of an exemplary information screen according to an embodiment of the invention;
**FIG. 2B** is an exemplary schematic representation of a substrate according to one embodiment of the invention;
**FIG. 2C** is a schematic of an exemplary information screen according to an embodiment of the invention;
**FIG. 2D** is a diagram of an exemplary probe array according to some embodiments of the invention;
**FIGS. 2E to 2H** are schematics of additional exemplary information screens according to embodiments of the invention;
**FIG. 3A** is a schematic diagram of registration of output data onto a pre-defined geometric model according to an exemplary embodiment of the invention;
**FIG. 3B** is a schematic diagram of a three dimensional substrate according to an embodiment of the invention;
**FIGS. 4A and 4B** are side views of a substrate depicting an exemplary probe and an exemplary substrate position indicator according to an exemplary embodiment of the invention;
**FIG. 5** is a simplified flow diagram of a method according to one exemplary embodiment of the invention; and
**FIG. 6** is a schematic of an exemplary user input interface according to one embodiment of the invention,
**FIGs. 7 to 10****, 12 and 13** are schematic of exemplary information screens according to embodiments of the invention;
**FIG. 11** is an exemplary set of user inputs devices according to one embodiment of the invention;
**Fig. 14** is a schematic representation of an exemplary graphical user interface according to an embodiment of the invention;
**Fig. 15** is a simplified flow diagram of a method according to an exemplary embodiment of the invention;
**Fig. 16** is a schematic representation of an exemplary supervision system according to some embodiments of the invention; and
**Fig. 17** is a schematic representation of one possible exemplary guidance system of Fig. 16 in greater detail.

### DETAILED DESCRIPTION OF EMBODIMENTS

### OVERVIEW

Various embodiments of the invention relate to computerized analytic systems, data acquisition tools for use in conjunction with the systems, and user interfaces adapted for data entry and/or data presentation. Specifically, some embodiments of the invention can be used to define and present individual data points in groups and/or to map data to a model of a substrate (region of interest) on which data points reside.

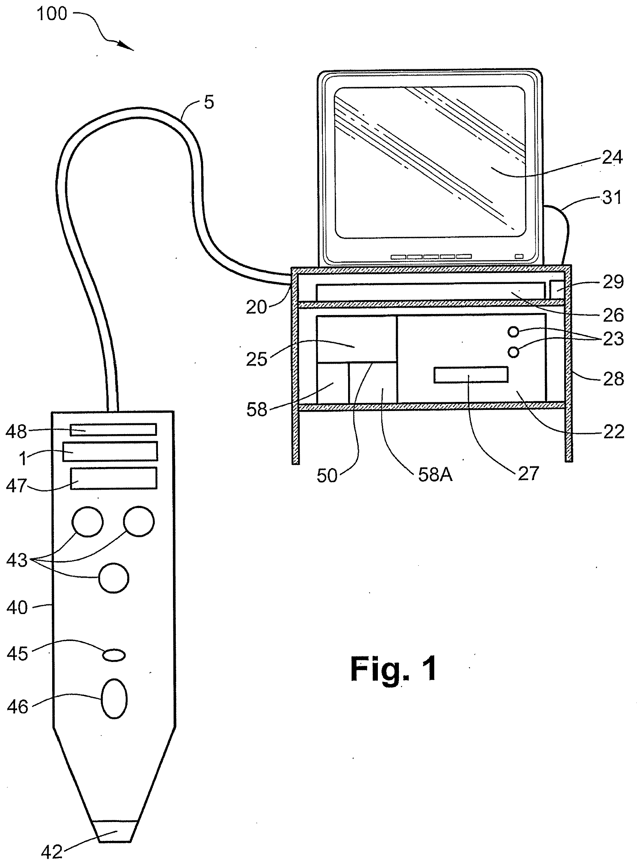

**Fig. 1** is a schematic diagram of an exemplary system **100** according to one embodiment of the invention. Depicted system **100** includes a data acquisition device or measurement unit (depicted as probe **40**). The probe **40** includes an operative portion **42** for scanning the substrate to be measured (contact or contactless scanning), and is configured and operable to measure and/or characterize one or more parameters indicative of one or more conditions or properties of the substrate in the scanned sites thereof.

In some exemplary embodiments of the invention, the substrate is a biological tissue, for example a piece of tissue excised from a subject during a surgical procedure. In other embodiments of the invention the substrate is a mineral substrate, for example a block of material removed from a quarry or mine.

According to various exemplary embodiments of the invention, probe 40 is configured to measure one or more of electromagnetic properties (e.g. using a nonirradiative sensor), dielectric properties, impedance, biological properties, chemical properties, optical properties (e.g. fluorescence emission and/or absorption and/or reflectance of selected wavelengths of light), MRI, energy transmission and/or reflectance (e.g. radio frequency [RF] or microwave [MW]) and temperature (e.g. via infrared thermography). Optionally, probe **40** is configured as a dielectric-property sensor, formed substantially as a coaxial cable.

In some exemplary embodiments of the invention probe **40** includes a single sensor at operative portion **42.** In other exemplary embodiments of the invention probe **40** includes an array of sensors at operative portion **42.**

In some exemplary embodiments of the invention, operative portion **42** includes a cutting tool and/or a sampling tool. Optionally, the cutting and/or sampling tool is separately controllable. In some exemplary embodiments of the invention, a controller operates the cutting and/or sampling tool responsive to data provided by the sensors.

Optionally, probe **40** includes or is associated with a control utility including *inter alia* one or more operative memory utilities **47,** a main database storage utility **48,** one or more control buttons **43** (e.g. for activating probe **40** and/or for initiating and/or measuring/characterization process, as well as a data processing utility (not shown here). Also, probe **40** may include one or more light sources such (depicted as LEDs **45** and **46**), an indicator or a display **1** (e.g. an LCD or CRT). In some exemplary embodiments of the invention, display **1** is used to display results and/or information pertinent to diagnosis and/or surgical planning. Optionally, results and/or information from one, two, three or more categories is presented.

There is a tradeoff between the amount of information presented on probe 40 and the ability of a user to organize and comprehend the information. In some exemplary embodiments of the invention, small amounts of information are transiently presented on probe 40 for immediate user evaluation and/or reaction and larger amounts of information are presented for longer periods of time on a display 24 which is physically separate from probe **40.**

**Fig. 1** depicts a data conduit **5** between probe **40** and an external control station **20.** Conduit **5** is drawn as a physical connection (e.g. wires or fiber optic cables) but could be replaced by a wireless link (e.g. Bluetooth, infrared, (IR) or radiofrequency (RF)).

Depicted control station or console **20** includes a control unit **22.** The latter is typically a computer system including *inter alia* one or more control buttons **23** that can be used, for example, to activate probe **40** and/or to initiate and/or control measuring and/or characterization. Optionally, control station **20** includes an additional input interface, such as a keyboard **26** or joystick **29** and/or a read/write device **27** (e.g. CD ROM or DVD drive) and/or a storage unit **50.** Storage unit **50** can include a data processor or processing unit. Optionally, control unit **22** may be configured to communicate with a signal analyzer **25** and/or an audio speaker **31** and/or a display screen **24.**

Control station **20** is depicted on a rack **28** although it could be incorporated into a hand-held device (e.g. personal digital assistant or smart-telephone) or a laptop computer.

Optionally, processing unit **50** includes a probe location module **58** and/or a physical marking module **58a.**

Exemplary probes and analytic systems useful in the context of various embodiments of the present invention, (e.g. for sensing, receiving, processing, storage and/or display) are described in U.S. Pat. No. 6,813,515 entitled "Method And System For Examining Tissue According To The Dielectric Properties Thereof" filed on January 4, 2002 and US Application No. 10/298,196 entitled "Method And Apparatus For Examining Tissue For Predefined Target Cells, Particularly Cancerous Cells, And A Probe Useful In Such Method And Apparatus" filed on November 18, 2002 and US Application No. 10/298,196 entitled "Clean Margin Assessment Tool" filed on March 23, 2005 and US Application No. 11/705,143 entitled "Methods, Systems, And Sensors For Examining Tissue According To The Electromagnetic Properties Thereof" filed on 12 February 2007, each of which are assigned to the common assignee of the present invention and each of which are hereby incorporated by reference.

Location module **58** and physical marking module **58a** optionally employ technology described, for example, in US 7,082,325 filed on July 15 2004 entitled "Method And Apparatus For Examining A Substance, Particularly Tissue, To Characterize Its Type".

In some exemplary embodiments of the invention, location module **58** relies upon medical imaging data. Medical imaging data includes, but is not limited to X-ray (e.g. fluorography or computerized tomography [CT]), magnetic resonance induction (MRI), ultrasound (US) and infrared (IR) imaging data.

Briefly, marking module **58a** issues a command to physically mark a measured location on the substrate in response to an instruction from processing unit **50.**

In some exemplary embodiments of the invention, the command causes deployment of a detectable material from operative portion **42** of probe **40** to physically mark a measurement location. Detection of the physical marking can be immediate or delayed by the user. Physical marking may be performed for example by using a visually detectable substance (e.g., one or more color biological marking ink, emitted from a jet nozzle mounted at operative portion **42** of probe **40.**

The processing unit analyzes a measurement from probe **40.** After substrate (e.g. tissue) recognition has been performed, marking module **58a** issues a color specific command to a jet nozzle (not pictured) and an appropriate color mark is printed on the substrate. According to some embodiments of the present invention, the one or more measurements in a region or area, e.g. which relate to the measurement outputs of the measured region or area, may be marked by using for example the marking module **58a.** The marking may correspond to the substrate characterization result/output and/or to region related data and/or to other data available from the processing unit **50.**

According to various embodiments of the invention, probe **40** may be configured as an extracorporeal device, an intracorporeal device, a device adapted for use on a portion of subcutaneous tissue, or a device adapted for use on a portion of an intracorporeal tissue during an open surgery.

Exemplary intracorporeal devices can be specifically configured for minimally invasive surgery and/or insertion via a trocar valve and/or for insertion via a body orifice to a body lumen (e.g. for use on a portion of inner lumen wall for further penetrating the lumen for use on a portion of an intracorporeal tissue outside the lumen) and/or for percutaneous insertion to a body lumen (e.g. for use on a portion of inner lumen wall for further penetrating the lumen for use on a portion of an intracorporeal tissue outside the lumen).

External display screen **24** and indicator **1** on probe **40** can be, for example CRT screens, LCD screens, plasma screens or projector displays or any other device capable of providing image or other data (e.g. audible output). In an exemplary embodiment of the invention, various categories of information are displayed in a user interface (e.g. Windows©, Linux© or Mac OS©). Optionally, multiple monitors may be used to display the data.

### EXEMPLARY GRAPHICAL USER INTERFACE

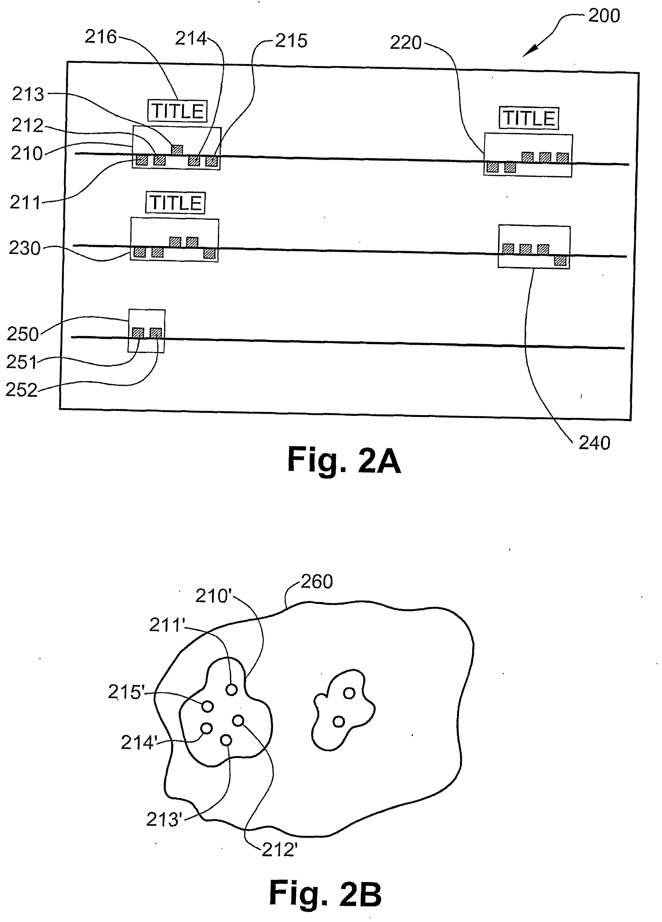

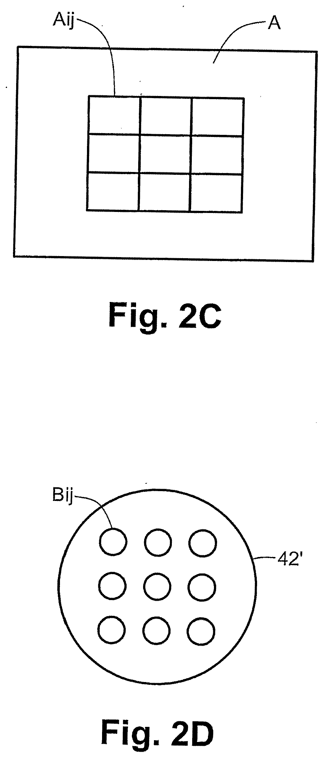

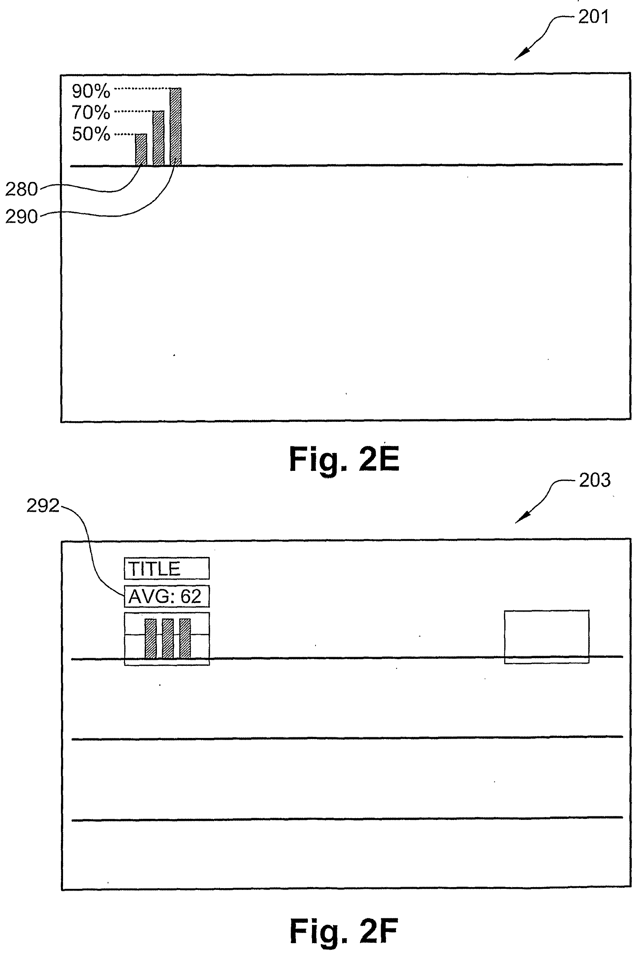

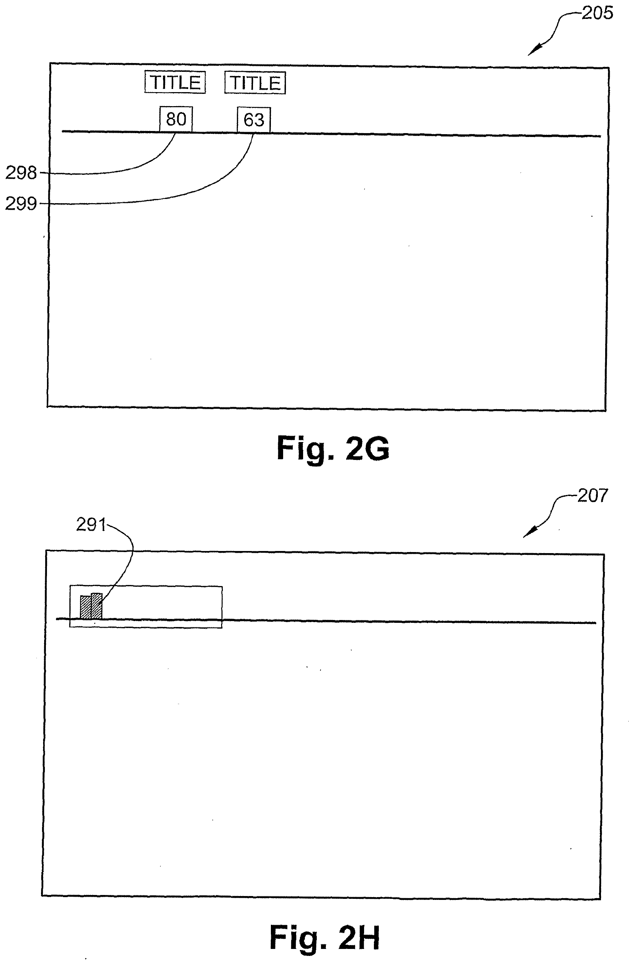

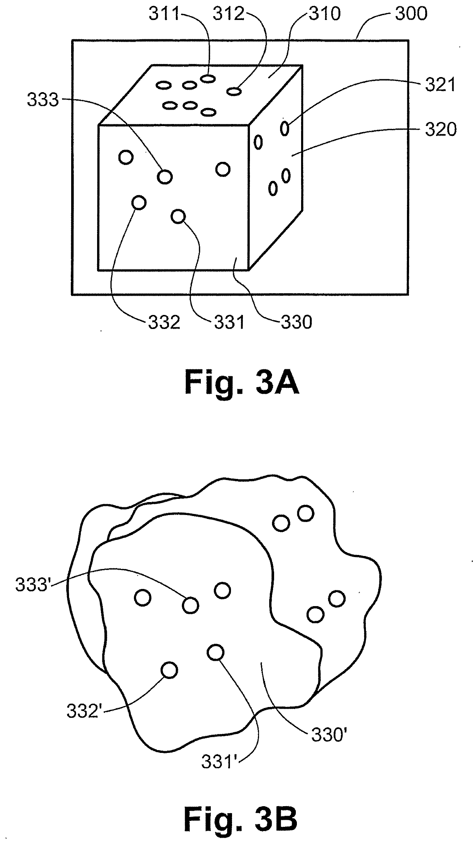

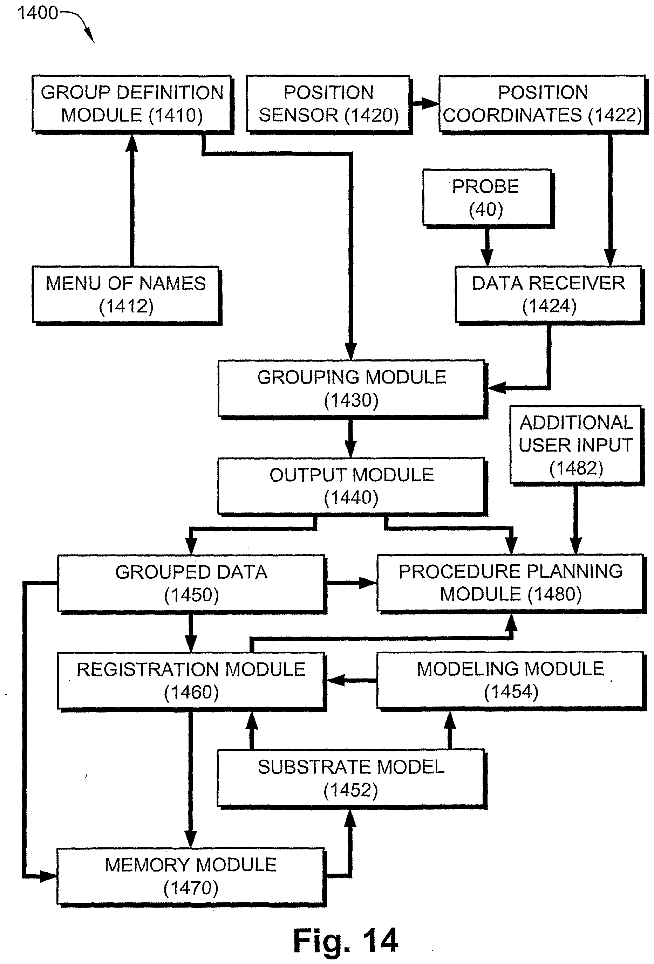

**Fig. 14** is a schematic block diagram illustrating interaction of components of exemplary graphical user interfaces (GUIs) **1400** according to some embodiments of the invention. **Figs. 2A****,** **2C****,** **2E, 2F****,** **2g, 2H****,** **3A and 3B** depict exemplary graphic output from a GUI of this type.

In some exemplary embodiments of the invention, GUI **1400** includes a group definition module 1410 adapted to accept a user input defining groups and a data receiver 1424 operable to receive a plurality of individual measurement input datum indicative of status of a substrate (e.g. from probe 40).

In some exemplary embodiments of the invention, the user input comprises a start command and a stop command. Optionally, the group definition module includes a single button and a first press of the button indicates "begin assigning measurement input datum to a group" and a second click of the button indicates "last measurement input datum received was the end of a group". In some embodiments of the invention, each click of the button after the first click indicates both an end of a preceding group and a beginning of a subsequent group. In some exemplary embodiments of the invention, the button is positioned on probe 40.

In some exemplary embodiments of the invention, the user input comprises a name. Optionally, the name can be selected from a menu (e.g. using a physical interface such as a scroll wheel, mouse or joystick) or entered manually by the user (e.g. using a keyboard or via a voice command).

Optionally, the name is indicative of a general location on the substrate. In some exemplary embodiments of the invention, group names are according to a commonly used convention among medical practitioners: Medial (M), Lateral (L), Posterior/Deep (D), Anterior/Superficial (SF), Inferior (I) and Superior (S). Optionally, multiple groups may be assigned to a same general location (e.g. S1, S2 or M1, M2 and M3).

In some exemplary embodiments of the invention, an interior surface of a body cavity serves as the substrate. Optionally, data collected from the cavity is used by append "C" or "CAV" to group names of the commonly used convention described above. Optionally, data is collected from an excised tissue and a cavity resulting from the excision in a single procedure.

Optionally, group numbers are used in place of names. In some exemplary embodiments of the invention, groups are successively numbered in their collection order.

In some exemplary embodiments of the invention, names are presented to a user in nested menus.

Optionally, a user selects a group name from a list of names, optional names descriptive of regions.

Optionally, the user enters names without using a list, for example by using keyboard 26 or buttons 43 on probe 40 the user may name region 210 'XL'. Alternatively or additionally, the user may store the name 'XL' in a 'language' database for example at database storage unit 48.

In some exemplary embodiments of the invention, groups are named by pressing control buttons 43 and 44, or the keyboard 26 and/or by a voice operating system or machine and/or by using a touch screen and/or a virtual touch screen (e.g., by relating movements and gestures of the surgeon hands for operations on the screen).

The user input defining groups and individual measurement input datum are processed by a grouping module 1430 configured to assign each of said individual measurement input datum to one of said groups to produce grouped data 1450 for output via an output module 1440.

Optionally, GUI 1400 includes a registration module 1460. In some exemplary embodiments of the invention, registration module 1460 registers grouped data 1450 onto a substrate model 1452. Registration can be based upon general positional information (e.g. as indicated by a group name) and/or on position coordinates for one or more points in a group. Substrate model 1452 can be, for example, a geometric solid representation of the substrate (e.g. a cube, sphere, cylinder, tube or polyhedral solid), a space filling model of the substrate (e.g. a model based upon general characteristics of an anatomic feature or organ being examined) or an image of the substrate produced using any known imaging technology. Optionally, registration to an image can be to a previously acquired image.

In some exemplary embodiments of the invention, individual datum indicative of status of said substrate are linked to position coordinates. Optionally, the position coordinates are defined relative to the substrate. One exemplary way to accomplish this is to use a position sensor 1420 to generate position coordinates 1422 to be transmitted to data receiver 1424 together with individual measurement input datum from probe 40. Optionally, position sensor 1420 is mounted on probe 40.

**Fig. 2A** is a schematic representation of an exemplary output 200 of grouped data 1450 from GUI 1400. Output 200 can be displayed, for example, on display screen 24 and/or indicator 1, e.g. the LCD and/or stored in a memory of processor 50 and/or written to a machine readable media in read/write drive 27. In some exemplary embodiments of the invention, output 200 displays information in a way designed to assist a user in making a diagnosis and/or planning a surgical response.

Depicted exemplary output 200 includes groups of measurements or results 210, 220, 230, 240 and 250. Optionally, each group corresponds to a region on the substrate. Groups may include one or more measurements or results and different groups can include different numbers of measurements/results.

For example region 210 includes five measurements outputs, e.g. measurements 211, 212, 213, 214 and 215, while region 250 includes two measurements outputs 251 and 252.

According to some embodiments of the invention, a single measurement relates to a single point on a substrate (e.g. a mass of excised tissue) and a group of measurements relates to an area or general location on the substrate where all the single measurements in the group were performed.

For example, as shown in **Fig. 2B****,** a user may examine an area 210' on substrate 260. The area 210' may be displayed in output 200 as group 210. The measurement output of each examined point in area 210', e.g. points 211', 212', 213', 214' and 215', may displayed, respectively, in region 210 for example as bars 211, 212, 213, 214 and 215 of Fig. 2A. Correlation between group 210 and area 210' is optionally provided by a descriptive title 216 or group name (e.g. L for lateral).

Optionally, each group is automatically named or numbered. For example the first region which was examined e.g. region 210 is numbered "One" and the following examined region is automatically numbered "Two". In some embodiments, the user may insert a title (e.g. name or number) to each region, for example according to the location of the region in the examined substance, or according to the number of examination.

According to some embodiments of the invention, a single measurement may be related to a single point on a substrate, such as a tissue, which was measured or characterized by a single sensor. Optionally, the sensor is one of a group of sensors provided in an array on operative portion 42 of probe 40.

According to other embodiments of the invention, a single measurement may be related to a plurality of points on a substrate measured by individual sensors belonging to a group of sensors provided in an array on operative portion 42 of probe 40. Optionally, the single measure is an arithmetic mean or median of a range of measurements. Optionally, an indication of variability is also provided.

In those embodiments of the invention which employ a group or array of sensors, the group or array is optionally operated in concert. Operation in concert refers to concurrent or sequential measurements performed by individual sensors in the group/array as a result of a single operation input or command. In some exemplary embodiments of the invention, use of groups or arrays of sensors contributes to an increase in accuracy and/or reliability of collected data without extending data collection time.

For example, as shown in **Fig. 2B****,** a surgeon may attach the probe 40 to the area 210' on substance 260. If operative portion 42 of probe 40 includes an array of sensors, each point in area 210' may be presented as a single datum indicative all measurements from all sensors in the array or as a series of data outputs (one output for each sensor in the array). Additionally, one or more measurement outputs of area 210' may be displayed on the information screen 200 using for example the display screen 24 or the indicator. 1, e.g. the LCD.

**Fig. 2C** depicts an exemplary matrix A of outputs of a sensor array 42' (**Fig. 2D**). In some exemplary embodiments of the invention a matrix A is presented for each of examined points 211', 212', 213', 214' and 215' in area 210'. According to these exemplary embodiments of the invention, individual measurements are displayed concurrently with each index *Aij* in matrix A representing respectively an output from a single measurement by the corresponding sensor *Bij* of sensor array 42'.

In other exemplary embodiments, measurements from sensor array 42' are condensed into a single datum (e.g. by averaging) for each of examined points 211', 212', 213', 214' and 215' in area 210'.

Alternatively or additionally, a data group (e.g. 210) includes multiple measurement outputs of Matrix A resulting from multiple operations of array 42' at multiple locations. For example the user may characterize a first substrate area (e.g. 211') using probe 40 with a sensor array 42' and a second substrate area (e.g. 214' or adjacent to the first area) by moving the senor array. For example, measurement outputs 211 of the first substrate area and 214 of the second substrate area are in the form of matrix A, and the group presentation 210 is a sequence of matrices A. Optionally, measurement outputs of the first substrate area and the second substrate area may be displayed as a single group (e.g. "M") or as separate groups (e.g. M1 and M2). Optionally, several probes including matrices are operated in parallel. In some exemplary embodiments of the invention, use of matrices reduces data collection time and/or contributes to an increase in meaningfulness of grouped data.

According to various embodiment of the invention, a single measurement output can be binary (e.g. yes/no or malignant/clear), discrete (e.g. on a scale with numbered units, optionally represented by symbols that increase in size incrementally) or continuous (e.g. a temperature, a conductivity, a color selected from a spectrum)
According to various embodiments of the present invention, a measurement output level or percentage of, for example a cancerous or non-cancerous substance (e.g. a substrate tissue) is displayed digitally (e.g. no/yes or red/green), or as a bar (e.g. the cancerous or non-cancerous level or percentage may be indicated according to a bar length, or color and/or position with respect to a baseline), a circle which is filled proportionally, as a combination of digital result and a bar, and only as a level result, e.g. including only the cancerous level without a yes or no indication.

Optionally, a faulty measurement e.g. faulty sensor signal, mechanical failure of the probe is indicated on the GUI, e.g. designated by an empty box and/or by a dedicated color and/or an error message.

**Fig. 2E** depicts an additional exemplary display screen 201 with bars 280 and 290 with lengths represent a cancerous percentage, (50% and 90% respectively) measured during a single measurement.

Optionally, individual measurement datum and/or group names are stored in system 100 (e.g. in control station 20 or in database storage unit 48 of probe 40).

According to some embodiments of the invention, each measurement datum acquired by probe 40 is summarized by a transient audio indication. For example, a beep may be used to indicate "normal" and a buzz may be used to indicate "abnormal" substrate status. A sequence of beeps may be used to indicate a faulty measurement

Alternatively or additionally, a status of grouping module 1430 may be indicated by audio signals. For example delivery of a "start" signal from group definition module 1410 can produce a single click which indicates that subsequently acquired individual datum will be grouped together and delivery of a "stop" signal from group definition module 1410 can produce a double click which indicates an end to grouping.

According to some embodiments of the present invention, each measurement datum acquired by probe 40 is summarized by a transient visual indication. For example, using the light sources 45 and 46 (e.g. red LED indicates abnormal measurement, green LED indicates normal measurement, yellow LED indicates a faulty measurement).

**Fig. 2F** depicts an additional exemplary display screen 203 featuring exemplary output of a summary statistic 292 (e.g. average) for grouped data 1450. Summary statistic 292 can be presented instead of, or in addition to, individual bars representing status indicative data from individual locations.

Types of summary statistic 292 include, but are not limited to mean average, mode, median, standard error of the mean (SEM) and standard deviation (SD). Optionally, two or more summary statistics are displayed concurrently (e.g. mean and SD or median, mean and SEM). Optionally, a total measurement output 292 for each region may be displayed separately. The total measurement output 292 represents the total of all the measurements performed in the region.

In some exemplary embodiments of the invention, summary statistic 292 indicates a number of datum in the group with a value exceeding a predefined threshold, a peak or an integral of all measurements in the group.

In some exemplary embodiments of the invention, summary statistic 292 is presented during data acquisition and is updated as new data is added to the group.

In some exemplary embodiments of the invention, summary statistic 292 is presented when data acquisition ends and the group is closed.

**Fig. 2G** depicts an additional exemplary display screen 205 showing measurements outputs 298 and 299 including an alpha numeric measurement indication.

**Fig. 2H** depicts concurrent display 291 of data from two different sensor types as described in detail hereinbelow.

As described hereinabove, output of grouped data 1450 comprises individual datum arranged in groups and/or at least one statistic summarizing the individual datum in each of the groups. Optionally, grouped data 1450 is stored in a memory module 1470.

In some exemplary embodiments of the invention, data receiver 1424 is adapted to receive individual measurement input datum concurrently from an array of probes. Alternatively or additionally, data receiver 1424 is adapted to receive individual measurement input datum concurrently from sensors of different types.

According to various exemplary embodiments of the invention, user input is provided at before and/or during and/or after receipt of said plurality of individual measurement input datum by data receiver 1424.

### EXEMPLARY APPARATUS FOR SUBSTRATE ANALYSIS

In some exemplary embodiments of the invention, system 100 (Fig. 1) includes an apparatus for analysis of a substrate. According to these embodiments of the invention, the apparatus includes probe 40 operable to produce a plurality of individual signal datum indicative of substrate status. Optionally, probe 40 is activated by contacting an operative portion 42 with a portion of the substrate and/or by operation of one or more control inputs 43. The apparatus includes group definition module 1410 (Fig. 14) adapted to accept a user input defining groups and grouping module 1430 configured to receive said signal datum indicative of substrate status and assign each of said individual datum to one of said groups to produce grouped data 1450 and an output module 1440 adapted to output grouped data 1450. According to various embodiments of the invention, output module 1440 includes a display (e.g. 1 and/or 24) and/or memory (e.g. 27 or 50) and/or printer (not depicted). In some embodiments of the invention, components such as group definition module 1410 and/or grouping module 1430 and/or output module 1440 are provided on probe 40.

In some exemplary embodiments of the invention, apparatus 100 includes one or more user input mechanism(s) on probe 40. In the embodiment depicted in Fig. 1, buttons 43 on probe 40 function as user input mechanisms.

Optionally, apparatus 100 includes at least one indicator on probe 40 (e.g. LEDs 45 and 46). These indicators function as part of output module 1440.

In some exemplary embodiments of the invention, probe 40 measures dielectric and/or electromagnetic properties or other properties of said substrate as described hereinabove. Probes capable of measuring dielectric and/or electromagnetic properties or other properties have been described and one of ordinary skill in the art will be capable of adapting them for use in the context of the invention.

In some exemplary embodiments of the invention, probe 40 is manually operable (e.g. by button press and/or by contact with substrate).

In some exemplary embodiments of the invention, a plurality of individual datum indicative of substrate status (e.g. 211, 212, 213, 214 and 215) is correlated to manually selected locations on the substrate (211', 212', 213', 214' and 215'; See Figs. 2A and 2B).

In some exemplary embodiments of the invention, the apparatus comprises a registration module 1460. Optionally, registration module 1460 registers the grouped data onto a model of the substrate. According the various embodiments of the invention, the model can be a solid representation of the substrate (e.g. a cube, sphere, dodecahedron or cylinder) and/or a space filling model of the substrate and/or an image of the substrate.

In some exemplary embodiments of the invention, user input is indicative of a location on said substrate. For example, group names entered via group definition module can be used to indicate which face of a cube grouped 1450 should be displayed upon.

**Fig. 3A** shows an exemplary output 300 of grouped data registered onto a three dimensional model. In the figure, the substrate is represented as a cube. Faces 310, 320 and 330 of the cube are visible. During examination of a substrate (e.g. an excised tissue mass) a user optionally selects an automatic region selection mode display. For example the user may measure substrate characteristics at points on different regions of the substrate (e.g. [311, 312] and [321] and [331,332 and 333]) where the square brackets indicate group designations supplied by the user via group definition module 1410.

In some exemplary embodiments of the invention, registration module 1460 applies grouped data 1450 to substrate model 1452 to produce an output of the type depicted in Fig. 3A. In the depicted exemplary output, substrate model 1452 is in the form of a cube, where each surface of the cube corresponds to a region of the cube according to the according to the M/L/D/SF/I/S nomenclature system described above.

In other exemplary embodiments of the invention, three dimensional shapes other than a cube are used as substrate model 1452 (e.g. sphere, dodecahedron, tube or cylinder.)

When shapes other than a cube are used, appropriate naming systems can be employed. For example, a spherical model can employ names based on longitude and/or latitude. A tubular or cylindrical model can employ names based on z (e.g. top, top-center, center, center- bottom, and bottom) and angle in plane perpendicular to z. For polyhedral shapes (e.g. dodecahedron) names can correspond to enumeration of faces in a predetermined order (e.g. 12 in the case of a dodecahedron), which may further be refined if the polyhedron has a specific symmetry (e.g. in the case of 6 face polyhedron: a cube, a box, a pyramid; each enable a more specialized enumeration)

**Fig. 3B** shows an irregularly shaped substrate with a surface 330' corresponding to face 330 of the cube model of Fig. 3A. Locations 331', 332' and 333' indicate sites of measurements 331, 332 and 333 respectively.

Although a simple substrate model 1452 is depicted in Fig. 3A, display 300 can include a model 1452 in the form of medical image data. Medical image date can include any imaging data, including image data types mentioned hereinabove. Optionally, the image data may is pre-acquired or acquired concurrent with acquisition of individual datum indicative of substrate status.

In some exemplary embodiments of the invention, individual datum indicative of substrate status belonging to a same group are displayed registered on substrate model 1452 with a sign (e.g. group indicator) or form (e.g. color) to aid the user in identifying which points belong to groups indicative of which substrate regions. Optionally, individual datum indicative of substrate status of all points in a first region may have a first sign or symbol such as a star and/or color such as orange and individual datum indicative of substrate status of all points which relate to a second region may include a second symbol such as a circle and/or a second color such as black. In some exemplary embodiments of the invention, the M/L/D/SF/I/S nomenclature is linked to specific symbols and/or colors. Optionally, the letters M/L/D/SF/I/S are used as symbols for the corresponding faces of the cube. Alternatively or additionally, colors are assigned to the six faces of the M/L/D/SF/I/S system (e.g. M-red, L-orange, D- yellow, SF- green, I- blue, S-violet).

Alternatively or additionally, each displayed individual datum indicative of substrate status includes data relating to substrate status using different formats as described in detail hereinabove. Alternatively or additionally, summary statistics 292 as described hereinabove are displayed on each face. Optionally, the summary statistics 292 summarize a singe group or all groups belonging to a particular face. Optionally, summary statistic is color coded or marked with a symbol as described above for individual datum.

According to some embodiments of the present invention individual datum indicative of substrate status belonging to a same group and/or residing on a same face are connected by a connector. Optionally, the connector is as a surface area connector enclosing all individual datum belonging to the group. Optionally, the connector is added automatically, for example by registration module 1460 and/or grouping module 1430. The connector can be, for example, a graphical indication of the relation between points. The graphical indication can include one or more of a line connecting the individual datum locations, a counter enclosing all the individual datum locations or a geometric shape contacting the individual datum locations. In some exemplary embodiments of the invention, the connector is dynamic, and is updated as additional points belonging to the group are added. Updating can be fully automatic or in response to an update command.

In some exemplary embodiments of the invention, individual datum indicative of substrate status are linked to position coordinates. Exemplary means of acquiring position coordinates are described hereinbelow.

Optionally, operative portion 42 of probe 40 includes a plurality of sub-probes arranged in a spatial array as described above with reference to Figs. 2C and 2D. In some exemplary embodiments of the invention, the sub-probes are collectively operable by a single probe activation signal. Optionally, contact with the substrate serves as a probe activation signal. In some exemplary embodiments of the invention, output of a group of sensors arranged in an array is used to provide a local summary of substrate status.

In some exemplary embodiments of the invention, probe 40 is operable in at least two modalities. For example, concurrent sensing and detection of electric impedance (EI) and magnetic resonance (MR) properties can be performed by operative portion 42 of probe 40. Optionally, sensors for two or more modalities are integrated into one sensing head.

In some exemplary embodiments of the invention, different sensing modalities are combined to produce a third "hybrid" modality. For example, concurrent measurement of EI properties of a specific region of the substrate and measurement of MR properties of the same substrate region produces a hybrid mode indicative of induced change in EI properties due to MR absorption of the incident electromagnetic radiation pulse.

### EXEMPLARY SUBSTRATE ANALYSIS METHOD

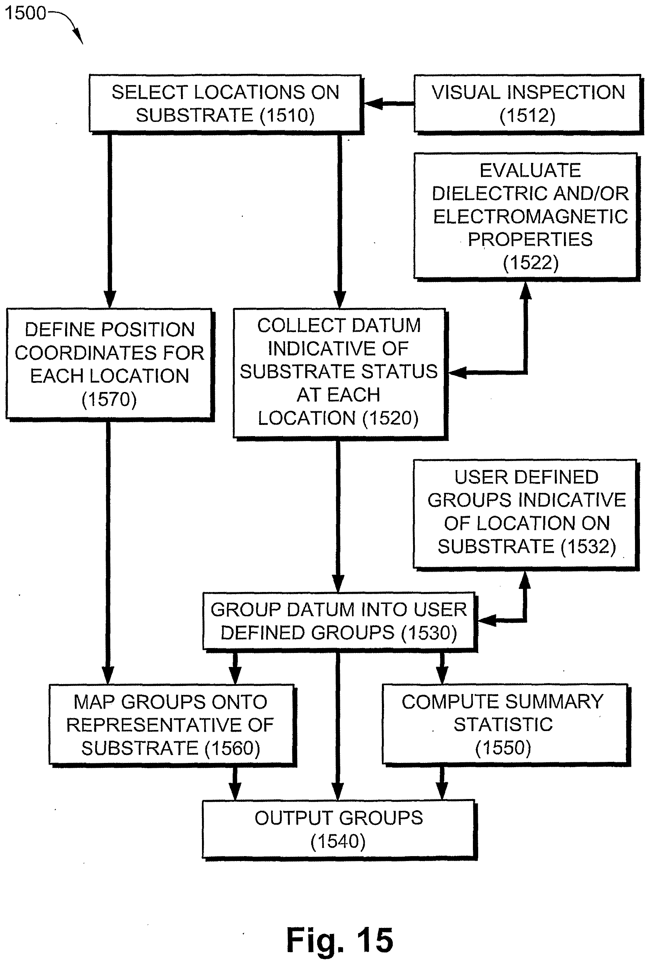

**Fig. 15** is a simplified flow diagram of an exemplary method 1500 of analyzing a substrate. Depicted exemplary method 1500 includes selecting 1510 a plurality of locations on a substrate collecting 1520 a datum indicative of substrate status at each location and grouping 1530 the data into groups defined by a user. Optionally, selecting 1510 is at least partially based upon visual inspection 1512 of the substrate by the user. According to some exemplary embodiments of the invention, datum indicative of substrate status reflect an evaluation 1522 of dielectric properties and/or electromagnetic properties of the substrate at each selected location.

According to method 1500, the user is presented with an output 1540 of the groups. In some exemplary embodiments of the invention, output of the data in groups contributes to an ability of the user to make a decision. Optionally, the decision relates to diagnosis and/or surgery. According to various embodiments of method 1500, output 1540 can be to a digital display (e.g. LCD or CRT screen) and/or printer and/or memory. The output can optionally include one or more additional features described below. Additional features can aid the user in making the decision.

In some exemplary embodiments of the invention, grouping 1530 is followed by computation 1550 of one or more summary statistics as described hereinabove. Optionally, the summary statistic is output 1540 in addition to, or as an indication of, group data.

In some exemplary embodiments of the invention, method 1500 includes mapping 1560 groups onto a representation of the substrate. Optionally, mapping 1560 is based upon definition 1570 of position coordinates for each location (as explained hereinbelow) and/or on user defined groups indicative of location 1532 (as explained hereinabove).

As explained hereinabove, mapping 1560 can be onto any representation of the substrate including but not limited to a polygonal solid representation of the substrate, a space filling model of the substrate and an image of the substrate.

### EXEMPLARY SYSTEM WITH MAPPING CAPABILITIES

In some exemplary embodiments of the invention, a substrate analysis system of the general type depicted in Fig. 1 as system 100 is modified with a position co-ordinate measurement module for determining position coordinates of the locations on the substrate.

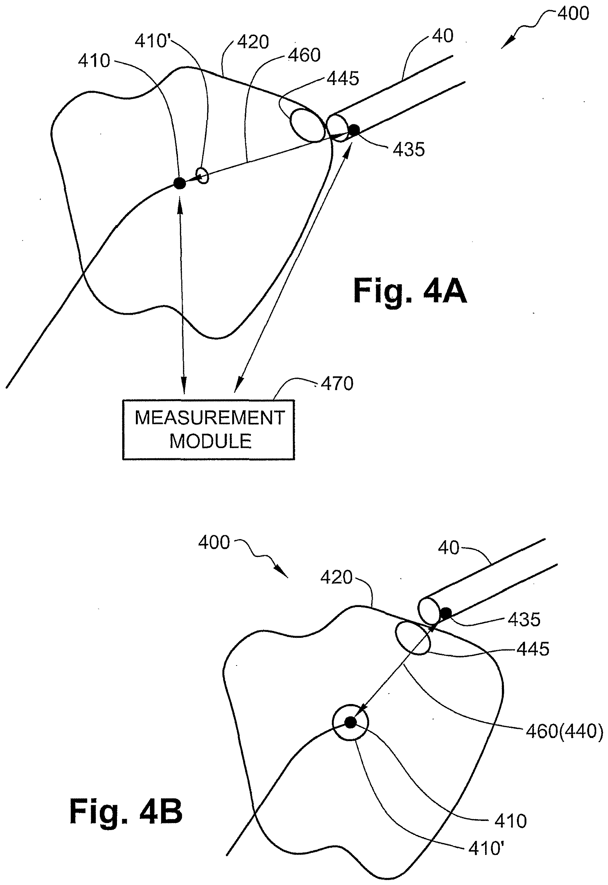

**Figs. 4A** and **4B** depict an exemplary measuring sub-system 400. Depicted exemplary measurement sub-system 400 employs a marker 410 in the substrate. Optionally, marker 410 has been implanted during a previous procure, such as image guided biopsy. Marker 410 can be passive or active.

Passive markers 410 can include, for example, a magnet or ultrasound reflector. Optionally, embodiments of the invention which include a passive marker 410 can include an active location detector 435 on probe 40 (e.g. a signal transducer).

Active markers 410 can include, for example, a radiofrequency RF or ultrasound (US) transducer. Optionally, embodiments of the invention which include an active marker 410 can include a passive marker 435 on probe 40.

In some exemplary embodiments of the invention, marker 410 is placed at or near a geometric center of substrate 420 (e.g. a tumor or excised tissue mass) indicated as 410'. It is often convenient to define 410' as an origin (0, 0, 0) for a three dimensional coordinate system defining locations on substrate 420.

In some exemplary embodiments of the invention (Fig. 4B), probe 40 includes an active sensor 435 adapted to measure a distance 460 and a direction, e.g. a relative vector (X,Y,Z) 440 between location 445 at which a substrate indicative datum is acquired and marker 410.

Fig. 4A depicts another embodiment of the invention in which positions of marker 410 in substrate 420 and marker 435 on probe 40 are each determined by a measurement module which determines a distance and direction 460 between measurement location 445 and center 410'.

According to one embodiment of the present invention, the probe 40 may include a structure, configured for receiving and holding a tissue specimen, wherein the tissue specimen includes tissue positional references and positional references, associated with the structure, for fixing the orientation of the tissue specimen, when held by the probe, so as to reflect the tissue specimen positional references. Such structure may be similar to various embodiments described, for example, in international publication number WO 2006/092797, entitled "Device And Method For Transporting And Handling Tissue", assigned to the common assignee of the present application and hereby incorporated by reference.

In some exemplary embodiments of the invention, system 100 includes a substrate position indicator 410 positioned at substrate 420 and a probe 40 operable to evaluate substrate status and output status indicative datum at individual points (e.g. 445) and measurement module 470 configured to determine a relative position of position indicator 410 and probe 40. In some exemplary embodiments of the invention, a controller (e.g. controller 22) coordinates operation of probe 40 and measurement module 470 so that said relative position is determined for each of said individual points. Optionally, a decrease in time difference between measurement of substrate status and of probe position contributes to an increase in accuracy of determined position for each substrate status measurement.

Optionally, the signal originates from probe 40 (e.g. from sensor 435) and is received at position indicator 410.

Optionally, the signal originates from position indicator 410 and is received at probe 40 (e.g. at sensor 435).

In some exemplary embodiments of the invention, measurement module 470 includes a position sensor adapted to determine a first position 445 of probe 40 (using marker 435) and a second position 410' indicating substrate location (using marker 410 to indicate (0,0,0)). According to these embodiments of the invention, measurement module 470 determines a relative position of marker 410 and marker 435 by comparing said first and second positions.

Other exemplary embodiments rely on any other technologies for determining relative position known to those of ordinary skill in the art.

Optionally, Measurement module is located external with respect to a patient body and/or with respect to substrate 420.

In some exemplary embodiments of the invention, system 100 includes an imaging module adapted to produce an image depicting said probe and said position indicator and measurement module 470 determines a relative position of probe 40 and substrate center 410' relative to image data. Optionally, the image data indicates markers 410 and/or 435.

In some embodiments of the invention, data indicative of substrate status is presented registered on a three dimensional representation of the substrate generated from the image data.

Optionally, probe 40 comprises a cutting tool. In some exemplary embodiments of the invention, the cutting tool is mounted at or near operative portion 42 and/or sensor 435 so that position and/or substrate status information for location 445 is relevant to the cutting tool.

In some exemplary embodiments of the invention, output module 1440 outputs relative position of probe 40 together with status indicative datum for each of individual points 445. Optionally, this contributes to an ability of registration module 1460 to register individual points 445 and/or grouped data 1450 on substrate model 1452.

Optionally, system 100 includes a procedure planning module 1480. Optionally, Procedure planning module 1480 is adapted to analyze said output from said output module and/or registration module 1460 and calculate a path. The calculated path optionally serves as a cutting path and/or a guide for delivery of implantable devices and/or medications. Exemplary implantable devices include, but are not limited to brachytherapy seeds and stents. Optionally, the path includes one or more location designations for performance of procedures (e.g. cutting and/or implantation, injection of medicine and/or ablation).

During the procedure planning (and/or guiding) actions other than cutting (e.g. navigation in consideration of anatomic features and/or administration of medication and/or implantation of devices) are optionally considered.

In some exemplary embodiments of the invention, planning module 1480 considers registration of status indicative data at multiple points 445 on substrate 420 registered to medical image data of any type when calculating a path. Alternatively or additionally, planning module 1480 is adapted to accept additional user input 1482 pertaining to a planned path (e.g. a cutting path) based upon output of said registration module. In some embodiments of the invention procedure planning module 1480 considers grouped data 1450 as well as outputs from output module 1440 and/or registration module 1460 inputs and outputs.

In some exemplary embodiments of the invention, planning module 1480 receives inputs from the user via an additional GUI. Optionally, the additional GUI includes a three dimensional graphical interface.

In some exemplary embodiments of the invention, planning module 1480 outputs a proposed path or other plan related information graphically, optionally as a three dimensional model. Optionally, graphic output of the proposed path or other plan related information is presented overlaid and/or registered with a medical image.

### EXEMPLARY ADAPTATION FOR SURGICAL SUPERVISION

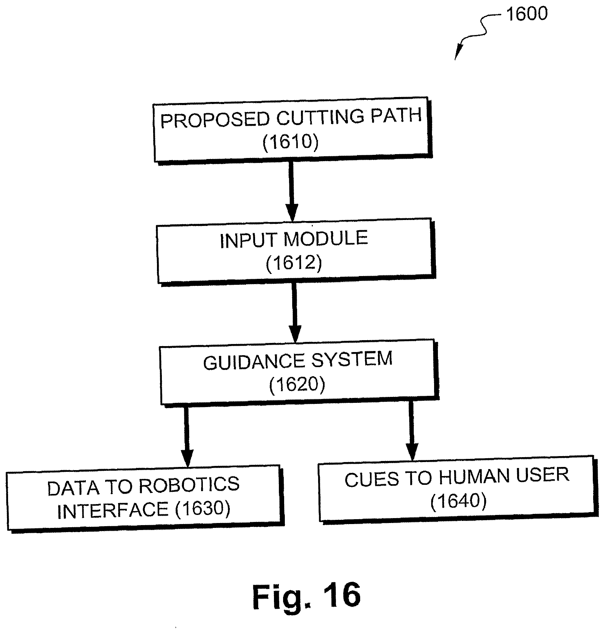

**Fig. 16** depicts a surgical supervision system 1600. Optionally, system 1600 aids in performance of surgery.

Depicted exemplary system 1600 includes an input module 1612 adapted to receive a proposed path (e.g. cutting) 1610 from a procedure planning module. The procedure planning module is optionally a planning module 1480 as described above. Depicted exemplary system 1600 also includes a guidance system 1620 adapted to output guidance instructions to guide a surgical tool along said path. Optionally, the instructions are provided in a data format 1630 suitable for use by a robotic interface and/or as cues 1640 suitable for use by a human user.

For example, display 300 (Fig. 3A) can include an image overlay such as probe reading (e.g. a binary reading per point) with a window showing an image such as a 3D image of the substrate and/or a characterized an anatomic feature. The reading optionally enables guiding of and/or instructions for guiding a surgical tool to a specific region shown on display 300. Optionally, probe readings are registered on the image.

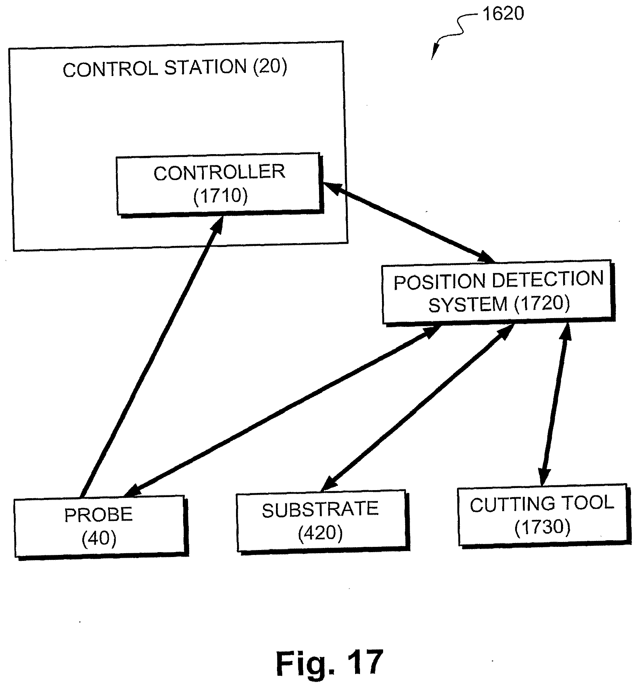

**FIG. 17** is a schematic representation of one possible configuration of a guidance system 1620 as shown in **Fig. 16** in greater detail. In the depicted exemplary configuration, position detection system 1720 determines positions of substrate 420 and probe 40 using any technology known in the art. Depending upon actual implementation, position detection system 1720 may rely upon markers implanted in substrate 420 and/or installed on probe 40 as described hereinabove.

In some exemplary embodiments of the invention a cutting tool 1730 is provided as a separate unit from probe 40. According to these exemplary embodiments, position detection system separately determines a position of cutting tool 1730.

In other exemplary embodiments of the invention cutting tool 1730 is provided as part of probe 40. According to these exemplary embodiments, position detection system concurrently determines the position of cutting tool and of probe 40.

In the depicted exemplary configuration, position detection system 1720 is in communication with a controller 1710, optionally integrated in control station 20.

In some exemplary embodiments of the invention, position detection system 1720 determines a relative position of probe 40 and/or cutting tool 1730 with respect to substrate 420.

In other exemplary embodiments of the invention, controller 1710 determines a relative position of probe 40 and/or cutting tool 1730 with respect to substrate 420 based upon position data received from position detection system 1720.

In the depicted exemplary configuration, controller 1710 also receives data indicative of substrate status as described hereinabove from probe 40. In some exemplary embodiments of the invention, controller 1710 integrates and/or correlates this data with corresponding position data for probe 40 to produce position correlated substrate status data. Optionally, controller 1710 constructs a status map from the position correlated substrate status data.

In some exemplary embodiments of the invention, controller 1710 compares the status map to the proposed path provided by input module 1612 (Fig. 16). As probe 40 and/or cutting tool 1730 are moved, the status map and/or proposed path can be updated by controller 1710 to produce a current status map and/or a current path.

In some exemplary embodiments of the invention, controller 1710 formulates data 1630 for the robotic interface and/or cues 1640 for the human user based upon the current status map and/or the current path.

### EXEMPLARY PROBE UNIT

Referring again to **Fig. 1****,** exemplary embodiments of the invention reside in substrate probe 40. Optionally, probe 40 is adapted to interact with system 100.

Depicted exemplary substrate probe 40 includes a data acquisition module adapted to engage a substrate at a user selected point, analyze the substrate and produce a datum indicative of substrate status at said point. The data acquisition module is depicted schematically as operative portion 42.

In some exemplary embodiments of the invention, operative portion 42 includes a pair of electrodes in communication with a cavity and a vacuum source configured to draw a portion of a substrate into the cavity so that it contacts the electrodes.

Depicted exemplary substrate probe 40 includes a user input device in the form of buttons 43 (three are depicted). In some exemplary embodiments of the invention, the user input device is configured to group individual datum into groups of data.

Optionally, fewer or more than three buttons 43 are provided. In some exemplary embodiments of the invention, a single button serves as a user input device. Optionally, buttons 43 are replaced by other mechanical, electronic or electromechanical input hardware (e.g. slider, rheostat, scroll wheel, microswitch).

Depicted exemplary substrate probe 40 includes a signal conduit 5 adapted to relay electrical and/or optical signals and a lumen adapted to relay a negative pressure from an external vacuum source to the data acquisition module (e.g. operative portion 42). In some exemplary embodiments of the invention, the lumen adapted to relay negative pressure resides in conduit 5. In other exemplary embodiments of the invention, the lumen adapted to relay negative pressure is provided separately.

Depicted exemplary substrate probe 40 includes a connector to an external data analysis component. Optionally, this connector resides in conduit 5 and/or relies upon a wireless link.

In some exemplary embodiments of the invention, probe 40 is provided as a sterile medical device. Optionally, probe 40 is provided in packaging configured to insure sterility until probe 40 is opened in an operating theater. Considerations in sterile packaging configuration are known to those of ordinary skill in the art of medical diagnostics and will be easily applied to embodiments of the invention.

In some exemplary embodiments of the invention, probe 40 comprises a user perceptible indicator. Optionally, the indicator provides a visible and/or audible indication as described above.

In order to make apparent some advantages of probe 40, an exemplary use scenario is described in detail.

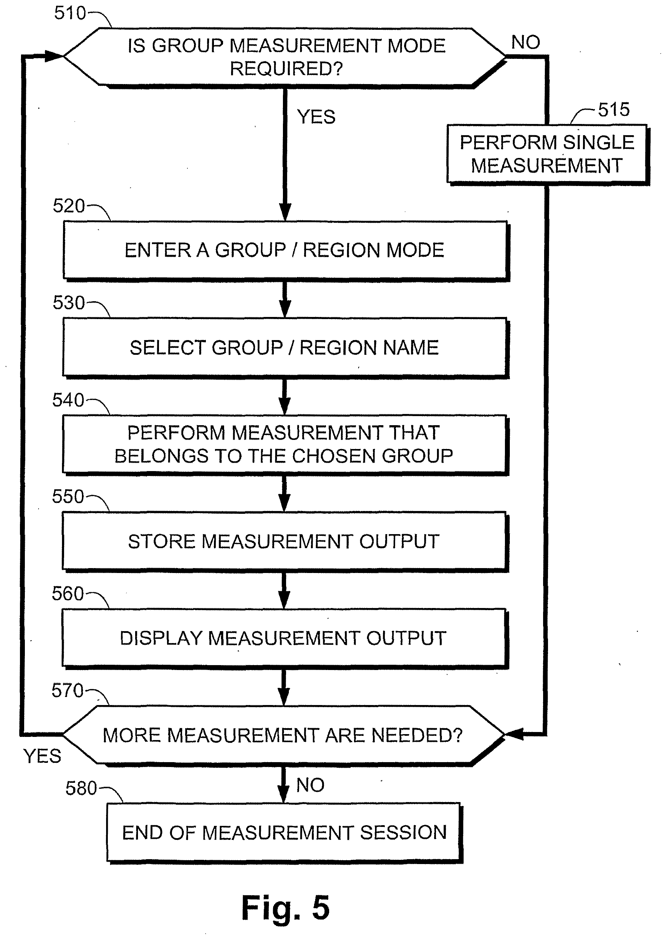

**Fig. 5** is an exemplary simplified schematic flow-chart of a method 500 for measuring and displaying measurements outputs of a substrate. At 510 a determination of whether group measurement mode is required is made.

If no, then at 515 a single measurement is performed, for example by probe 40.

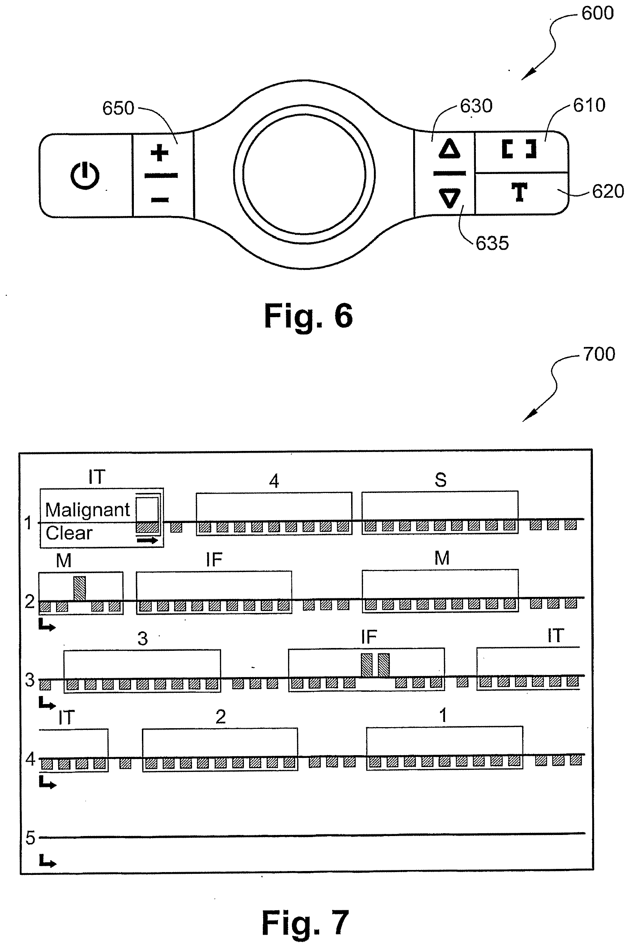

If yes, then at 520 a group mode is entered and a group/region name is entered or selected from a predefined list at 530. Entry can optionally be by a user (e.g. physician) or automatic as described hereinbelow in relation to Fig. 6.

At 540 one or more measurements which belong to the chosen group are performed, for example by probe 40.

At 550 measurement outputs are stored. Storage can be, for example in control station 20 or in database storage unit 48 of probe 40.

At 560 measurement outputs are displayed. Display is optionally on display screen 24 or indicator 1.

At 570 a determination of whether more measurements are necessary is made.

If yes, return to 510 with probe 40 being moved to a new point and/or substrate region.

If no, end measurement session at 580.

### EXEMPLARY DATA ENTRY INTERFACE

**Fig. 6** is schematic diagram of an exemplary embodiment of front panel buttons 600 of console 20.

According to some embodiments of the invention, operations done using the console front panel buttons 600 may alternatively be performed using button(s) 43 of probe 40.

For example, a double click on button 43 may initiate group mode process. Optionally, a consecutive number, starting for example with '1', is automatically presented in response to the double click. Alternatively, letter can be employed in a predefined order, for example according to M/L/D/SF/I/S convention.

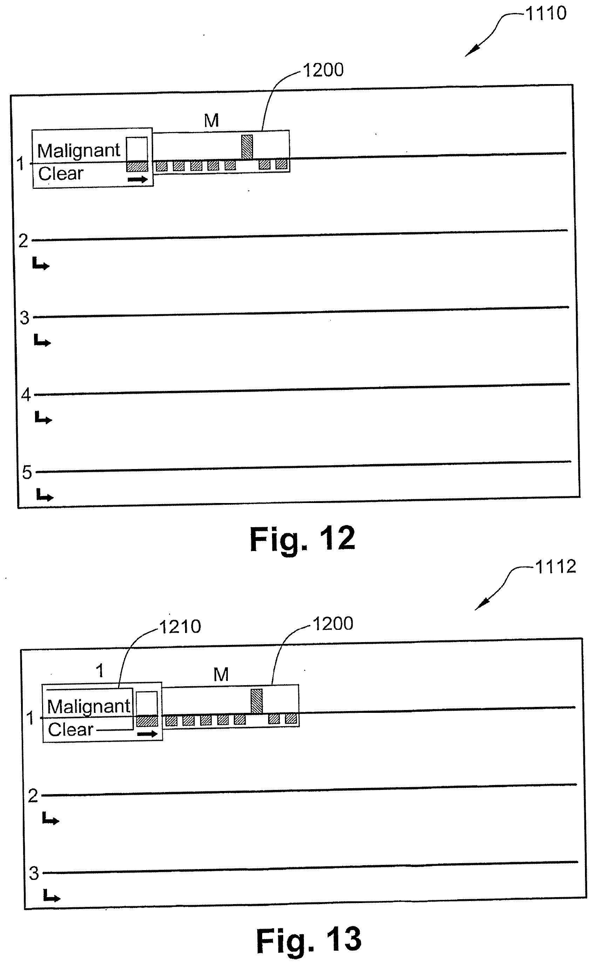

**Fig. 12** depicts an exemplary display screen 1110 in which a second double click optionally closes a group mode, for example depicted first group 1200 (M). Optionally, closing of a group closes a frame around the group as shown.

**Fig. 13** depicts an exemplary display screen 1112 in which an additional double click opens a second group 1210 (1) as shown. Optionally, opening of a group causes a new frame to appear. Optionally the new frame is open on its left side as depicted.

Alternatively, button 610 on panel 600 (e.g. of console 20) can be operated in an analogous manner to perform the same functions.

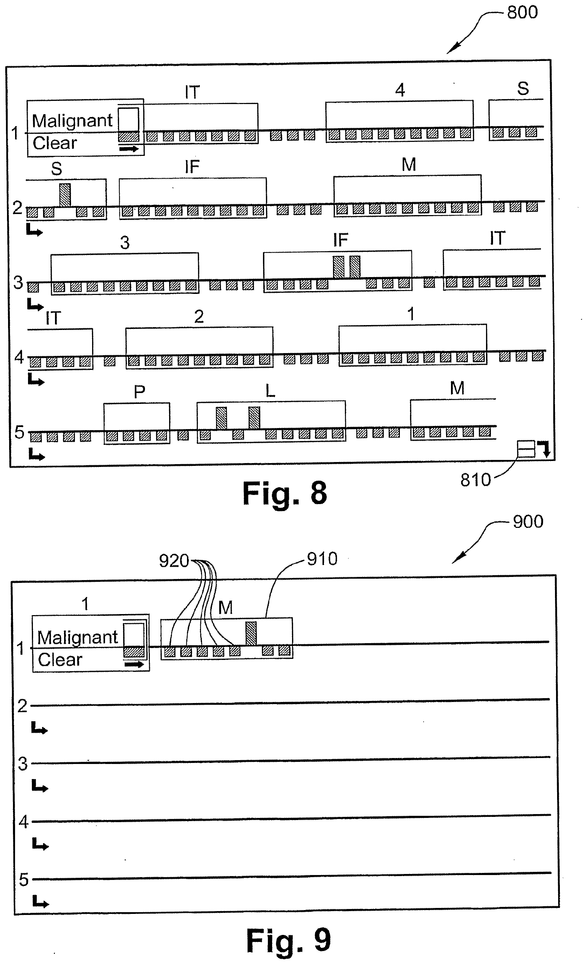

**Fig. 9** depicts an exemplary display screen 900 in which a "closed" group 910 (optionally representative of a region [e.g. M] on the substrate) including one or more measurements outputs 920 is illustrated. According to some embodiments of the present invention, a measurement may also be initiated immediately, without entering a group mode. A long single press on the button 43 of probe 40 or button 620 on console 600 enters a group name mode. Optionally, a menu of names is accessed (e.g. Medial - M, Lateral-L, Posterior (Deep)-D, Anterior (Superficial)-SF, Inferior-I, Superior-S, # - number that was automatically chosen for the group, CAV - to enter into relevant sides of the cavity).

In some embodiments each short press on probe 40 button 43 or alternatively, by pressing on either button 630 or button 635 on console front panel 600 presents a different group name, for example in cyclic consecutive order.

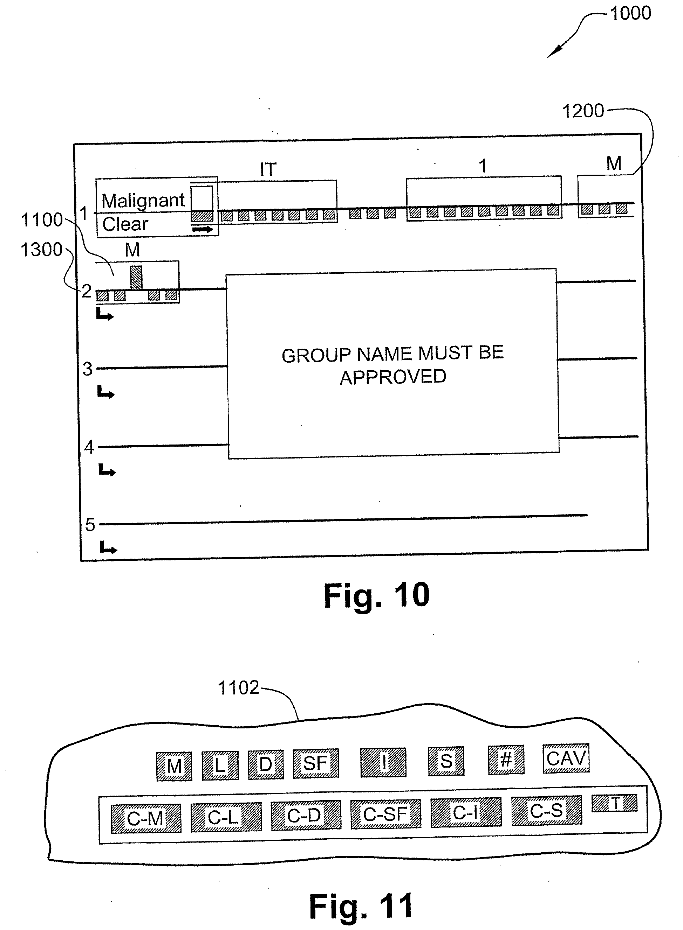

**Fig. 11** depicts an exemplary set of user inputs (e.g. buttons) 1102. According to some embodiments of the invention buttons 1102 may be provided in the control station 20 or probe 40. Optionally, buttons are labeled with group names corresponding to region names as indicated. Alternatively or additionally, a similar set of names can be presented as a menu on a display screen.

According to some embodiments of the present invention, the group to be presented after a first press will be the next group that follows after the previous chosen group. A sign '#' may represent a group number which was automatically chosen by the system. Once the region or group name is approved (e.g. by a long press on button 43 or 620), the user may scroll between a list of regions (e.g. using button 650). A long press on button 43 or 620 approves a specific region as indicated by group name. In case one of the region options was chosen, entering group name mode for next group will start immediately with a list of regions options. A group's name may be approved by a long press on probe's button 43 or by pressing on button 620 on console's front panel 600.

**Fig. 10** depicts an exemplary display screen 1000 According to some embodiments of the present invention, a group name may be colored, for example in red until it is approved by long button press on button 43 or 620. Once approved, the color of the group's name may be switched for example to yellow. In some embodiments measurements can not be taken until group is approved. Optionally, a group name may be changed, even after it was approved, as long as the group was not closed. In some embodiments after a group was closed, the group name can not be changed. In some embodiments, actions can not be done before approving a group name. For example a message, such as an audio or digital message, will pop up on the screen in cases such as: group name was not approved, and/or after two seconds from choosing a name.

To exit group mode a user may double press on button 43 or 610 on the console. Optionally, the user may open a second group by double clicking button 43. According to some embodiments, the measurements can be performed immediately without choosing group name. In case a group name was not chosen it could be chosen at any time, for example as long as the group mode is active. In some embodiments a group name may be chosen more than one time.

**Fig. 8** illustrates an exemplary embodiment of a display screen 800 in which a output of a next measurement "pushes" previous measurements to a next row which is not visible on the screen, and a scroll indicator 810 is activated, as shown in Fig. 8. The user may use button 43 or button 640 or the joystick for scrolling up or down the measurements.

According to some embodiments of the present invention, when a screen line or row is full with measurements outputs or region output the "next" measurement output of the same region output or the next single measurement output may be presented in the "next" row. For example, as shown in Fig. 10, the next measurement output 1100 of region output 1200 is displayed in the next row 1300.

An error massage may be displayed on probe 40 or on display screen 24. In some embodiments an indication of an error message may be activated using for example an audio indicator machine.

A group may be selected before the measurements related to that group have been initiated. Alternatively, the group may be selected after some of the measurements which related to the group have been acquired or alternatively the group may be selected after all the measurements related to the group have been acquired.

**Fig. 7** depicts an exemplary output screen 700 in which current measurements are highlighted. Highlighting can be, for example, by changing characteristics of background and/or a frame indicating a current group and/or characteristics of a current measurement (e.g. bar width or color).

According to some embodiments of the present invention, data from a session is saved in storage unit 48 for further analysis. Optionally, session data may also be exported and transmitted to an external device/computer or to an external memory device such as a removable memory e.g. a DiskonKey or other small and portable memory device. Session data recorded or saved may be used, for example, in additional procedures, such as pathology procedures, related to the examined tissue. Session data which was recorded or saved may be also used in additional procedures, such as additional surgical or diagnostic procedures on a same subject.

### EXEMPLARY ANALYSIS WITH REGISTRATION

Referring again to **Fig. 14****,** additional exemplary embodiments of the invention which are independent of data grouping are described.

Some exemplary embodiments of the invention are of an apparatus 1400 for analysis of a substrate including a probe 40 as described hereinabove, a modeling module 1454 adapted to produce a three dimensional model of the substrate and a registration module 1460 adapted to indicate the specified locations on the model. Optionally, the modeling module relies upon defined position coordinates 1422 and/or substrate models 1452 (e.g. predefined geometric models or realistic modes indicative of actual substrate shape or models based on medical image data).

Optionally, the apparatus includes an output module adapted to present an indication of substrate status at each of the specified locations on the model. In some exemplary embodiments of the invention, presentation is as an overlay. In some exemplary embodiments of the invention, the model is a 3D model which can be manipulated (e.g. rotated or inverted) during viewing.

In some exemplary embodiments, apparatus 1400 is adapted to accept a user input specifying a position of said locations (e.g. user provides coordinates 1422).

In other exemplary embodiments, apparatus 1400 is adapted to determine a position of said locations. Optionally, apparatus 1400 employs position sensors 1420 adapted to determine a position of said locations.

Optionally, output of registered data from registration module 1460 is to one or more of a graphic display, a printer and a memory.

It is expected that during the life of this patent many substrate measurement techniques and medical imaging methods will be developed and the scope of the invention is intended to include all such new substrate measurement techniques and medical imaging methods *a priori.*

## Claims

1. A substrate analysis system, the system comprising:
(a) a probe configured and operable for scanning a substrate and determine one or more parameters indicative of one or more conditions or properties of the substrate at individual points corresponding to the probe position, and thereby evaluating substrate status at said individual points and generating output status indicative datum for said individual points; the system being **characterized in that** it comprises:
(b) a substrate position indicator configured for positioning at the substrate being analyzed;
(c) a measurement module configured for receiving signals from at least one of the substrate position indicator and the probe, and determining a relative position of said position indicator and said probe; and
(d) a controller adapted to coordinate operation of said probe and said measurement module so that said relative position is determined for each of said individual points.

2. A substrate analysis system according to claim 1, comprising a position sensor adapted to determine a first probe position and a second substrate position indicator position; wherein said measurement module is adapted to determine said relative position by comparing said first and second positions.

3. A substrate analysis system according to claim 1 or 2, comprising an imaging module adapted to produce an image depicting said probe and said position indicator; wherein said measurement module is adapted to determine said relative position from said image.

4. A substrate analysis system according to any of claims 1 to 3, comprising a registration module adapted to register said status indicative datum from said individual points with medical image data.

5. A substrate analysis system according to any of claims 1 to 3, comprising a registration module adapted to register local summaries of status indicative datum with medical image data.

6. A substrate analysis system according to any of claims 1 to 5, comprising a cutting tool mounted on said probe.

7. A substrate analysis system according to any of claims 1 to 6, comprising an output module adapted to output said relative position together with said status indicative datum for each of said individual points.

8. A substrate analysis system according to claim 7, comprising a procedure planning module which is adapted for receiving and analyzing said output from said output module and to calculate a path.

9. A substrate analysis system according to claim 8, wherein said procedure planning module is adapted to accept user input pertaining to a planned path.

10. A substrate analysis system according to claim 8 or 9, wherein said procedure planning module is connected to a guidance system to use data generated by the procedure planning module about a path to generate output guidance instructions to guide a surgical tool in accordance with said path.

11. A substrate analysis system according to any one of claims 1 to 11, wherein said probe comprises:
(a) a data acquisition module adapted to engage a substrate at a user selected point, analyze the substrate and produce a datum indicative of substrate status at said individual point;
(b) a position determination module adapted to provide a position of said individual point;
(c) a user input device;
(d) a signal conduit adapted to relay electrical signals; and
(e) a lumen adapted to relay a negative pressure from an external vacuum source to said data acquisition module.

12. A substrate analysis system according to claim 11, wherein said position determination module is adapted to provide position coordinates defined relative to said substrate.

13. A substrate analysis system according to claim 11 or 12, wherein the user input device of the probe is adapted to group said datum into groups of data.

14. A substrate analysis system according to any one of claims 11 to 13, wherein the probe comprises a indicator of said substrate status.

15. A controller for use in a substrate analysis system, the controller comprising:
a probe location module for receiving data from a substrate probe comprising status indicative datum at individual points,
a measurement module comprising a position sensor for receiving data from a substrate position indicator positioned at said substrate, and providing measured data indicative of a relative position of said position indicator and said substrate probe, and
a position detection system for processing the measured data and generating operation data to the substrate probe and to the measurement module to coordinate their operation to provide determination of said relative position for each of said individual points.

## Patentansprüche

1. Substratanalysesystem, wobei das System umfasst:
(a) eine Sonde, die konfiguriert und betriebsfähig ist, um ein Substrat abzutasten und einen oder mehrere Parameter zu bestimmen, der bzw. die eine oder mehrere Bedingungen oder Eigenschaften des Substrats an einzelnen Punkten, die der Sondenposition entsprechen, bestimmt bzw. bestimmen, und um dadurch einen Substratstatus an den einzelnen Punkten zu bewerten und ein einen Ausgangsstatus angebendes Datenelement für die einzelnen Punkte zu generieren; wobei das System **dadurch gekennzeichnet ist, dass** es umfasst:
(b) einen Substratpositionsindikator, der konfiguriert ist, um an dem analysierten Substrat positioniert zu sein;
(c) ein Messmodul, das konfiguriert ist, um Signale von mindestens einem von dem Substratpositionsindikator und der Sonde zu empfangen, und um eine relative Position des Positionsindikators und der Sonde zu bestimmen; und
(d) einen Controller, der geeignet ist, um die Betätigung der Sonde und des Messmoduls derart zu koordinieren, dass die relative Position für jeden der einzelnen Punkte bestimmt wird.

2. Substratanalysesystem nach Anspruch 1, umfassend einen Positionssensor, der geeignet ist, um eine erste Position der Sonde und eine zweite Position des Substratpositionsindikators zu bestimmen; wobei das Messmodul geeignet ist, um die relative Position durch Vergleichen der ersten und zweiten Positionen zu bestimmen.

3. Substratanalysesystem nach Anspruch 1 oder 2, umfassend ein Bildgebungsmodul, das geeignet ist, um ein Bild zu erzeugen, das die Sonde und den Positionsindikator darstellt; wobei das Messmodul geeignet ist, um die relative Position aus dem Bild zu bestimmen.

4. Substratanalysesystem nach einem der Ansprüche 1 bis 3, umfassend ein Registrierungsmodul, das geeignet ist, um das einen Status angebende Datenelement von den einzelnen Punkten mit medizinischen Bilddaten zu registrieren.

5. Substratanalysesystem nach einem der Ansprüche 1 bis 3, umfassend ein Registrierungsmodul, das geeignet ist, um lokale Zusammenfassungen eines einen Status angebenden Datenelements mit medizinischen Bilddaten zu registrieren.

6. Substratanalysesystem nach einem der Ansprüche 1 bis 5, umfassend ein Schneidwerkzeug, das an der Sonde montiert ist.

7. Substratanalysesystem nach einem der Ansprüche 1 bis 6, umfassend ein Ausgabemodul, das geeignet ist, um die relative Position zusammen mit dem einen Status angebenden Datenelement für jeden der einzelnen Punkte auszugeben.

8. Substratanalysesystem nach Anspruch 7, umfassend ein Arbeitsablaufplanungsmodul, das geeignet ist, um die Ausgabe von dem Ausgabemodul zu empfangen und zu analysieren und um eine Bahn zu berechnen.

9. Substratanalysesystem nach Anspruch 8, wobei das Arbeitsablaufplanungsmodul geeignet ist, um eine Benutzereingabe anzunehmen, die eine geplante Bahn betrifft.

10. Substratanalysesystem nach Anspruch 8 oder 9, wobei das Arbeitsablaufplanungsmodul mit einem Führungssystem verbunden ist, um Daten über eine Bahn, die durch das Arbeitsablaufplanungsmodul generiert werden, zu verwenden, um Ausgangsführungsanweisungen zu generieren, um ein chirurgisches Instrument gemäß der Bahn zu führen.

11. Substratanalysesystem nach einem der Ansprüche 1 bis 11, wobei die Sonde umfasst:
(a) ein Datenerfassungsmodul, das geeignet ist, um ein Substrat an einem vom einem Benutzer ausgewählten Punkt in Eingriff zu bringen, das Substrat zu analysieren, und ein Datenelement zu erzeugen, das einen Substratstatus an dem einzelnen Punkt angibt;
(b) ein Positionsbestimmungsmodul, das geeignet ist, um eine Position des einzelnen Punkts bereitzustellen;
(c) eine Benutzereingabevorrichtung;
(d) eine Signalleitung, die geeignet ist, um elektrische Signale weiterzuleiten; und
(e) ein Lumen, das geeignet ist, um einen Unterdruck von einer externen Vakuumquelle an das Datenerfassungsmodul weiterzuleiten.

12. Substratanalysesystem nach Anspruch 11, wobei das Positionsbestimmungsmodul geeignet ist, um Positionskoordinaten bereitzustellen, die mit Bezug auf das Substrat definiert sind.

13. Substratanalysesystem nach Anspruch 11 oder 12, wobei die Benutzereingabevorrichtung der Sonde geeignet ist, um das Datenelement in Datengruppen zusammenzufassen.

14. Substratanalysesystem nach einem der Ansprüche 11 bis 13, wobei die Sonde einen Indikator des Substratstatus umfasst.

15. Controller zur Verwendung in einem Substratanalysesystem, wobei der Controller umfasst:
ein Sondenstandortmodul, um von einer Substratsonde Daten, die ein einen Status angebendes Datenelement an einzelnen Punkten umfassen, zu empfangen,
ein Messmodul, das einen Positionssensor umfasst, um Daten von einem Substratpositionsindikator, der an dem Substrat positioniert ist, zu empfangen, und um gemessene Daten, die eine relative Position des Positionsindikators und der Substratsonde angeben, bereitzustellen, und
ein Positionsdetektionssystem, um die gemessenen Daten zu verarbeiten und Betätigungsdaten für die Substratsonde und das Messmodul zu generieren, um ihre Betätigung zu koordinieren, um eine Bestimmung der relativen Position für jeden der einzelnen Punkte bereitzustellen.

## Revendications

1. Système d'analyse de substrat, le système comprenant :
(a) une sonde configurée et actionnable pour balayer un substrat et déterminer un ou plusieurs paramètres indiquant une ou plusieurs conditions ou propriétés du substrat à des points individuels correspondant à la position de sonde et, de ce fait, évaluer un état de substrat auxdits points individuels et générer une donnée d'indication d'état de sortie pour lesdits points individuels ; le système étant **caractérisé par le fait qu'**il comprend :
(b) un indicateur de position de substrat configuré pour se positionner au substrat en cours d'analyse ;
(c) un module de mesure configuré pour recevoir des signaux provenant d'au moins l'un parmi l'indicateur de position de substrat et la sonde, et déterminer une position relative dudit indicateur de position et de ladite sonde ; et
(d) un dispositif de commande adapté pour coordonner le fonctionnement de ladite sonde et dudit module de mesure de telle sorte que ladite position relative est déterminée pour chacun desdits points individuels.

2. Système d'analyse de substrat selon la revendication 1, comprenant un capteur de position adapté pour déterminer une première position de sonde et une seconde position d'indicateur de position de substrat ; ledit module de mesure étant adapté pour déterminer ladite position relative par comparaison desdites première et seconde positions.

3. Système d'analyse de substrat selon la revendication 1 ou 2, comprenant un module d'imagerie adapté pour produire une image représentant ladite sonde et ledit indicateur de position ; ledit module de mesure étant adapté pour déterminer ladite position relative à partir de ladite image.

4. Système d'analyse de substrat selon l'une quelconque des revendications 1 à 3, comprenant un module d'enregistrement adapté pour enregistrer ladite donnée d'indication d'état à partir desdits points individuels avec des données d'image médicale.

5. Système d'analyse de substrat selon l'une quelconque des revendications 1 à 3, comprenant un module d'enregistrement adapté pour enregistrer des résumés locaux de donnée d'indication d'état avec des données d'image médicale.

6. Système d'analyse de substrat selon l'une quelconque des revendications 1 à 5, comprenant un outil de coupe monté sur ladite sonde.

7. Système d'analyse de substrat selon l'une quelconque des revendications 1 à 6, comprenant un module de sortie adapté pour délivrer en sortie ladite position relative conjointement avec ladite donnée d'indication d'état pour chacun desdits points individuels.

8. Système d'analyse de substrat selon la revendication 7, comprenant un module de planification de procédure qui est adapté pour recevoir et analyser ladite sortie provenant dudit module de sortie, et pour calculer une trajectoire.

9. Système d'analyse de substrat selon la revendication 8, dans lequel ledit module de planification de procédure est adapté pour accepter une entrée d'utilisateur relative à une trajectoire planifiée.

10. Système d'analyse de substrat selon la revendication 8 ou 9, dans lequel ledit module de planification de procédure est connecté à un système de guidage pour utiliser des données générées par le module de planification de procédure concernant une trajectoire pour générer des instructions de guidage de sortie afin de guider un outil chirurgical conformément à ladite trajectoire.

11. Système d'analyse de substrat selon l'une quelconque des revendications 1 à 11, dans lequel ladite sonde comprend :
(a) un module d'acquisition de données adapté pour engager un substrat au niveau d'un point sélectionné par l'utilisateur, analyser le substrat et produire une donnée indiquant un état de substrat audit point individuel ;
(b) un module de détermination de position adapté pour fournir une position dudit point individuel ;
(c) un dispositif d'entrée d'utilisateur ;
(d) un conduit de signal adapté pour relayer des signaux électriques ; et
(e) une lumière adaptée pour relayer une pression négative d'une source de vide externe audit module d'acquisition de données.

12. Système d'analyse de substrat selon la revendication 11, dans lequel ledit module de détermination de position est adapté pour fournir des coordonnées de position définies par rapport audit substrat.

13. Système d'analyse de substrat selon la revendication 11 ou 12, dans lequel le dispositif d'entrée d'utilisateur de la sonde est adapté pour regrouper ladite donnée en groupes de données.

14. Système d'analyse de substrat selon l'une quelconque des revendications 11 à 13, dans lequel la sonde comprend un indicateur dudit état de substrat.

15. Dispositif de commande pour une utilisation dans un système d'analyse de substrat, le dispositif de commande comprenant :
un module de localisation de sonde pour recevoir des données provenant d'une sonde de substrat comprenant une donnée d'indication d'état à des points individuels,
un module de mesure comprenant un capteur de position pour recevoir des données provenant d'un indicateur de position de substrat positionné audit substrat, et fournir des données mesurées indiquant une position relative dudit indicateur de position et de ladite sonde de substrat, et
un système de détection de position pour traiter les données mesurées et générer des données de fonctionnement pour la sonde de substrat et pour le module de mesure pour coordonner leur fonctionnement afin de permettre la détermination de ladite position relative pour chacun desdits points individuels.
